# EUROPÄISCHE PATENTSCHRIFT

(11) **EP 3 522 833 B1**
(45) Veröffentlichungstag und Bekanntmachung des Hinweises auf die Patenterteilung: **06.08.2025**
(21) Anmeldenummer: 17769090.6
(22) Anmeldetag: 20.09.2017
(51) Int. Cl.: A61F 2/50, A61F 2/64, A61F 2/68

(54) **PROTHESENKOSMETIK UND SYSTEM AUS PROTHESENKOSMETIK UND PROTHESE**
PROSTHESIS COSMETIC ELEMENT, AND SYSTEM CONSISTING OF PROSTHESIS COSMETIC ELEMENT AND PROSTHESIS
RECOUVREMENT ESTHÉTIQUE DE PROTHÈSE ET SYSTÈME CONSTITUÉ D'UN RECOUVREMENT ESTHÉTIQUES DE PROTHÈSE ET D'UNE PROTHÈSE

(30) Priorität: 06.10.2016 DE 102016119001
(43) Veröffentlichungstag der Anmeldung: 14.08.2019
(73) Patentinhaber: Otto Bock Healthcare Products GmbH, 1110 Wien (AT)
(72) Erfinder: PILLER, Markus, 1110 Wien (AT); OPPEL, Hans, 1090 Wien (AT); WAGNER, Sonja, 1180 Wien (AT); BISCHOF, Johannes, 2700 Wiener Neustadt (AT); FREY, Alice, 2000 Stockerau (AT); SCHEFFEL, Raphael, 1100 Wien (AT); LUNZER, Walter, 1150 Wien (AT); KOPPE, Mario, 37085 Göttingen (DE); LEINIGER, Andreas, 37327 Leinefeld OT Birkungen (DE)
(74) Vertreter: Gramm, Lins & Partner Patent- und Rechtsanwälte PartGmbB
(86) Internationale Anmeldenummer: PCT/EP2017/073787
(87) Internationale Veröffentlichungsnummer: WO 2018/065218

(56) Entgegenhaltungen:
- DE-A1- 102012 009 757
- DE-B3- 102014 015 756
- DE-U1- 202007 009 077
- US-A1- 2007 150 069

## Beschreibung

Die Erfindung betrifft ein System aus einer Prothesenkosmetik und einer Prothese mit einem Prothesengelenk mit einem Oberteil und einem schwenkbar um eine Schwenkachse daran gelagerten Unterteil. Die Prothesenkosmetik ist insbesondere zur Verkleidung von Prothesen der unteren Extremität geeignet, insbesondere zur Verkleidung von Prothesenbeinen bei Patienten mit einem Oberschenkelstumpf. Grundsätzlich kann eine solche Prothesenkosmetik auch an einer oberen Extremität angeordnet sein, insbesondere bei Patienten mit einem Oberarmstumpf und einem Prothesenellenbogengelenk.

Prothesen dienen als Ersatz für nicht vorhandene oder nicht mehr vorhandene natürliche Gliedmaßen und sollen in erster Linie eine nicht vorhandene oder verlorengegangene Funktionalität zumindest teilweise wiederherstellen. Daneben existieren reine Schmuckprothesen, die das Fehlen einer natürlichen Gliedmaße kaschieren. Prothesen mit einem Prothesengelenk weisen Einrichtungen zur Festlegung des Prothesengelenkes an dem Stumpf auf, mit denen das Oberteil des Prothesengelenkes über einen Prothesenschaft an dem Stumpf festgelegt wird. Hierzu existieren verschiedene Möglichkeiten. Schwenkbar an dem Oberteil ist ein Unterteil angeordnet, an dem weitere Prothesenkomponenten befestigt sein können. Beispielsweise können Dämpfereinrichtungen, Aktuatoren oder weitere Prothesenkomponenten, wie Unterschenkelrohre, Prothesenhände oder Prothesenfüße daran festgelegt sein. Die Gestaltung dieser Prothesenkomponenten erfolgt in erster Linie aufgrund funktioneller Aspekte, die Materialien sollen möglichst haltbar, belastungsgerecht und leicht sein. Dies führt in der Regel dazu, dass die Erscheinungsform einer Prothese eher technisch ist.

Um das äußere Erscheinungsbild einer Prothese an das Erscheinungsbild der nicht vorhandenen Gliedmaße anzunähern, werden sogenannte Prothesenkosmetiken an der Prothese angeordnet, die das äußere Erscheinungsbild der Prothese prägen. Die Prothesenkosmetik kann aus unterschiedlichen Materialien bestehen und kann auch technische Funktionen übernehmen, beispielsweise Schutz vor mechanischen Einwirkungen, vor Umwelteinflüssen sowie in eingeschränktem Maße eine Bereitstellung von Rückstellkräften, sofern die Kosmetik während der Bewegung der Prothese verformt wurde. Als Material der Prothesenkosmetik wird häufig Schaumstoff eingesetzt, der entsprechend der Außenkontur der zu ersetzenden Gliedmaße moduliert werden muss. Über den Schaumstoff kann ein Überzug aus einem Elastomer gezogen werden, um eine hautähnliche Erscheinungsform nachzubilden. Die Schaumstoffe sind somit mit sogenannten Kosmetikhäuten beschichtet, die nach Anwärmen auf die Schaumstoffgrundform durch Aufschrumpfen aufgebracht oder aufgeklebt werden müssen.

Aus der WO 2010/054341 A1 ist ein Verfahren zum Herstellen von individuell gestalteten Vorrichtungen oder Prothesenverkleidungen bekannt, bei dem Referenzpunkte an dem Körper markiert werden. Eine Vielzahl von Bildern wird aus einer Vielzahl von Winkeln aufgenommen und verwendet, um die Kontur des Körpers aufzunehmen. Die anderen Markierungen werden aufgebracht und verwendet, um die Kosmetik herzustellen. Die Kosmetik kann als einteilige Konstruktion oder als mehrteiliges Produkt ausgebildet sein, das montiert werden muss.

Die US 5,880,964 A betrifft ein System und Verfahren zum Herstellen einer Prothesenkosmetik, bei dem die Prothese an dem Nutzer angelegt wird. In einer Datenbank sind die Daten sowohl des Prothesenschaftes als auch der Prothesenkomponenten abgelegt. Diese Daten werden verwendet, um die Abmessungen der inneren Oberfläche der Kosmetik zu berechnen. Über CAD-Systeme wird eine Außenkontur über ein automatisches Schleifverfahren hergestellt, die der Kontur der Gliedmaße auf der unversorgten Seite entspricht.

Die US 6,597,965 A betrifft ein Verfahren zur Herstellung einer Schaumkosmetik für eine prothetische Komponente. Auf der Grundlage der Daten der prothetischen Komponente werden zwei Hälften einer inneren Kavität errechnet. Eine computergesteuerte Schleifmaschine schneidet einen Teil der einen Hälfte der Kavität in eine Seite des Blockes und einen Teil der anderen Hälfte auf die gegenüberliegende Seite des Blockes. Der Block wird halbiert, die einander gegenüberliegenden Hälften werden miteinander verbunden, so dass der Block die Prothesenkomponente umschließt und die Prothesenkomponente in der zusammengefügten Kavität aufgenommen wird.

Die US 6,740,124 A betrifft eine Endoprothese für Oberschenkelamputierte mit einem Schaft, einem schaumabgedeckten Pylon und einem Prothesenfuß. Eine Kunststoffumhüllung wird geformt, erwärmt und gedehnt, bis es eine Form aufweist, mit der die Hülle den Schaumstoff umgeben kann. In den Bereichen mangelhafter Passform wird die Hülle mit Heißluft erwärmt.

Die US 8,366,789 B1 betrifft eine Protheseneinrichtung mit einer äußeren Oberfläche, die spiegelbildlich zur Oberfläche des gesunden Beines ausgebildet ist. Die gesunde Gliedmaße wird eingescannt und die erhaltenen Daten werden verarbeitet, um eine entsprechende Kontur zu erhalten. Die Designdaten der Protheseneinrichtungen werden im Rahmen des Rapid-Prototyping verarbeitet und eine Kosmetik hergestellt. Die Prothesenkosmetik erstreckt sich über den Schaft und das Prothesengelenk.

Die DE 20 309 318 U1 betrifft einen Kniebereichsteilungsadapter zur lösbaren Verbindung eines ersten kosmetischen Schaumteils, das über ein Kniegelenk einer Beinprothese schiebbar und über einen Oberschenkelschaft stülpbar ist. Ein zweites kosmetisches Schaumteil ist um ein Unterschenkelteil der Beinprothese herum angeordnet. Zwei miteinander lösbar verbindbare, plattenförmige Adapterteile sind vorgesehen, wobei ein erstes Adapterteil mit dem ersten kosmetischen Schaumteil und ein zweites Adapterteil mit dem zweiten kosmetischen Schaumteil lösbar verbindbar ist. Das Schaumteil umgibt das Prothesengelenk vollständig.

Die US 5,376,127 A betrifft eine leichtgewichtige Prothesenkosmetik mit einer Kontur und Erscheinung einer menschlichen Gliedmaße. Die Kosmetik ist um eine Prothesenkomponente herum angeordnet und durch Erwärmen und Umformen eines vorgefertigten geschlossenzelligen Polyethylen-Plattenmaterials hergestellt.

Die US 2007/0150069 A1 betrifft eine modularisierte Beinprothesenkosmetik mit einem Oberschenkelmodul, das an einem Oberschenkelschaft festlegbar ist, und einem Unterschenkelmodul, das an einem Unterschenkelrohr festlegbar ist sowie einem Patellamodul, das schwenkbar mit dem Unterschenkelmodul verbunden ist. Die Kosmetik, die durch Befestigung der Module aneinander erhalten wird, wird über ein Hautmodul abgedeckt, das aus einer dehnbaren Faser hergestellt ist.

Die US 2007/0162154 A1 betrifft eine orthopädische Abdeckung für ein Prothesenteil mit einem hohlen, im Wesentlichen zylindrischen Grundkörper zur Aufnahme der prothetischen Komponente, mit einer Öffnung, die ausreichend groß ist, damit ein Prothesenkniegelenk eingebeugt werden kann. Die Breite ist ausreichend bemessen, um Schutz für die mechanischen Komponenten des Prothesenkniegelenkes bereitzustellen.

Die DE 10 2012 009 757 A1 betrifft eine Protheseneinrichtung und eine Verkleidung für eine Protheseneinrichtung, bei der die äußere Kontur der Protheseneinrichtung individuell anpassbar ist. Ein Drehmodul wird eingesetzt, um einen unteren Teil relativ gegenüber einem oberen Teil eines ersten Teils einer Protheseneinrichtung um deren Längsachse zu verdrehen. Dadurch soll im verdrehten Zustand eine ästhetische Verkleidung bewirkt werden.

Die DE 20 2007 009 077 U1 kann als nächstliegender Stand der Technik angesehen werden und betrifft eine Beinprothese aus einem Unterteil mit einem daran angeordneten Fußteil und einem Oberschenkel, das kniegelenkartig an dem Unterschenkelteil angelenkt ist. Das Oberschenkelteil ist von einem Kniegelenk bis zu einem Ende des Stumpfes mit einer Verkleidung auf Silikonbasis versehen, die in etwa chronisch von dem Stumpf bis zu dem Kniegelenk zuläuft. Das Unterschenkelteil ist von einer wadenähnlichen Abdeckung umgeben.

Problematisch bei den Prothesenkosmetiken aus dem Stand der Technik ist häufig, dass sie nicht über den Gelenkbereich hinausgehen und somit auch keine zwischen dem Oberteil und dem Unterteil stattfindende Relativbewegung abdecken. Reine Schaumstoffumhüllungen sind anfällig gegen Verschmutzung und stellen einen Widerstand bei Beuge- oder Streckvorgängen der Gelenke durch die Materialfestigkeit und die elastischen Eigenschaften dar. Darüber hinaus gibt es Schwierigkeiten, die Schaumstoffüberzüge schnell auszuziehen oder anzuziehen oder gegebenenfalls zu wechseln.

Aufgabe der vorliegenden Erfindung ist es, ein System aus einer Prothesenkosmetik und einer Prothese bereitzustellen, bei der eine Abdeckung über den Gelenkbereich hinweg möglich ist, ohne wesentliche Einschränkungen bei der Handhabbarkeit der Prothese in Kauf nehmen zu müssen.

Erfindungsgemäß wird diese Aufgabe durch ein System mit den Merkmalen des Hauptanspruches gelöst. Vorteilhafte Weiterbildungen und Varianten der Erfindung sind in den Unteransprüchen, der Beschreibung sowie den Figuren offenbart.

Das erfindungsgemäße System aus einer Prothesenkosmetik und einer Prothese mit einem Prothesengelenk, das ein Oberteil und einem schwenkbar daran um eine Schwenkachse gelagerten Unterteil aufweist, sieht vor, dass die Prothesenkosmetik ein erstes Rahmenteil aufweist, das zumindest eine Befestigungseinrichtung zur Festlegung an dem Unterteil aufweist. Darüber hinaus weist die Prothesenkosmetik ein zweites Rahmenteil auf, das zumindest eine Befestigungseinrichtung zur Festlegung an dem Oberteil aufweist, wobei die beiden Rahmenteile verschwenkbar zueinander gelagert sind. An dem Prothesengelenk und bevorzugt an einem Rahmenteil ist ein Drehelement insbesondere in Gestalt eines Drehadapters befestigt, wobei dieser über ein Betätigungselement verriegelbar oder entriegelbar ausgebildet ist. Das Betätigungselement ist von außen mit einem Rahmenteil oder durch ein Rahmenteil hindurch zugänglich. Das Betätigungselement öffnet oder schließt eine Sperreinrichtung oder eine Verriegelungseinrichtung innerhalb des Drehadapters, so dass von außen im angelegten Zustand mit einem Textilüberzug versehen, eine Veränderung der Ausrichtung der Prothesenkosmetik über eine Verdrehung und/oder Verschwenkung eines Teils des Rahmens relativ zu einem anderen, insbesondere relativ zu einem Kopfbereich vorgenommen werden kann. Sofern innerhalb der Prothesenkosmetik Einstellungen an der Prothese vorgenommen werden sollen, ist es vorteilhafterweise vorgesehen, dass an dem Rahmenteil zumindest eine Ausnehmung oder zumindest ein Betätigungselement angeordnet sind, durch die von außen eine Einstellung vorgenommen werden kann.

Das Drehelement kann von zumindest einem Rahmenteil zumindest teilweise umgeben sein, wobei das Drehelement durch das zumindest eine Rahmenteil hindurch oder mit dem zumindest einem Rahmenteil betätigbar ist. Dazu ist bevorzugt das Drehelement an einem der beiden Rahmenteile befestigt. Insbesondere kann das Drehelement an dem Oberteil und/oder dem zweiten Rahmenteil befestigt sein, um die Zugänglichkeit und sichere Zuordnung zu erleichtern.

Eine Weitebildung der Erfindung sieht vor, dass zumindest eines der beiden Rahmenteile einen zumindest teilkugelartig oder teiltonnenartig ausgebildeten Kopfbereich aufweist, der in dem anderen Rahmenteil drehbar gelagert ist. Die Prothesenkosmetik ist insbesondere für Prothesenellenbogengelenke oder Prothesenkniegelenke vorgesehen und weist einen Rahmen mit im Gelenkbereich kugelförmigen oder teilkugelförmigen bzw. tonnenförmigen oder teiltonnenförmigen Kopfbereichen auf, so dass auch in einem eingebeugten Zustand eine der natürlichen Erscheinungsformen angenäherte Kontur bereitgestellt wird. Die Rahmenteile bilden einen Unterbau, auf dem weitere Prothesenkosmetikkomponenten angeordnet sein können. Das jeweilige Rahmenteil wird an dem Oberteil oder Unterteil des Prothesenkniegelenkes festgelegt oder an weiteren Komponenten des Prothesengelenkes montiert, so dass die Bewegung des Oberteils bzw. des Unterteils mit dem jeweiligen Rahmenteil ebenfalls aufgeführt wird. Der Kopfbereich des ersten Rahmenteils an dem Unterteil ist proximal ausgebildet, ein Kopfbereich des zweiten Rahmenteils ist distal ausgebildet. Bevorzugt steht der Kopfbereich des ersten Rahmenteils proximal über die Schwenkachse über. In dem Rahmenteil sind funktionswesentliche Komponenten des Unterteils oder der sich an dem Unterteil anschließenden prothetischen Komponenten angeordnet, beispielsweise ein Unterschenkelrohr, eine Dämpfereinrichtung oder ein Aktuator. Der Rahmen lässt Platz für obere und untere Anschlussmittel, beispielsweise ein Pyramidenadapter oder Schraubanschluss für ein Unterschenkelrohr, Unterarmrohr oder einen Prothesenschaft. Eine solche Anbindung bietet die Möglichkeit für eine kosmetische Überbrückung der Gelenkanbindung und dem Schaft.

Eine Weiterbildung der Erfindung sieht vor, dass an beiden Rahmenteilen, also sowohl an dem ersten Rahmenteil als auch an dem zweiten Rahmenteil, ein teilkugelartiger oder teiltonnenartiger Kopfbereich ausgebildet ist, die in einer gestreckten Stellung des Gelenkes einander zumindest teilweise überdecken, wobei ein Kopfbereich in dem anderen Kopfbereich aufgenommen ist. So ergibt sich in der gestreckten Stellung zumindest teilweise eine doppelte Überdeckung der prothetischen Gelenkeinrichtung, so dass ein besonders guter Schutz der Protheseneinrichtung in Verbindung mit einer natürlichen Erscheinungsform erreicht werden kann.

In einer Weiterbildung der Erfindung ist vorgesehen, dass in einer eingebeugten Stellung des Prothesengelenkes die Kopfbereiche einander ergänzend das Prothesengelenk zumindest teilweise umgeben. Bei einem Prothesenkniegelenk sind die Kopfbereiche bevorzugt so angeordnet und dimensioniert, dass in einer maximal flektierten Stellung die Kopfbereiche einander noch überdecken oder bündig miteinander abschließen. Ziel ist es, möglichst keinen Spalt zuzulassen, der Zugang zwischen den beiden Rahmenteilen zu der dahinterliegenden Gelenkeinrichtung zulässt. Die Abdeckung ist dabei frontal geschlossen, so dass eine Einbeugung weiterhin möglich ist. Durch diese Ausgestaltung wird verhindert, dass ein kosmetischer Überzug, der über die Rahmenteile angelegt wird, bei der Bewegung des Prothesengelenkes eingeklemmt wird. Entsprechendes gilt bei einer Anwendung bei einem Prothesenellenbogengelenk.

Das erste Rahmenteil kann schalenartig oder mit in Längserstreckung des Unterteils ausgerichteten Streben ausgebildet sein. Bei einer schalenartigen Ausgestaltung des ersten Rahmenteils ist parallel oder längs zu dem Unterteil und der sich daran anschließenden Protheseneinrichtung, beispielsweise einem Unterschenkelrohr oder einer prothetischen Gelenkeinrichtung mit einer Mechanik, Hydraulik oder einem Aktuator, eine ganzflächige Abdeckung im Bereich der schalenartigen Abdeckung ausgebildet, um einerseits eine glattflächige Kontur und andererseits einen hohen mechanischen Schutz bereitzustellen. Durch die schalenartige oder rinnenartige Ausgestaltung, die die Frontseite beispielsweise eines Unterschenkelrohrs abdeckt, kann einerseits eine Kontur der natürlichen Gliedmaße nachgebildet und anderseits ein mechanischer Schutz bereitgestellt werden.

Alternativ zu einer schalenartigen und damit flächigen Abdeckung ist in einer Variante vorgesehen, dass zumindest zwei Streben oder Streifen sich in Längserstreckung des Unterteils bzw. der sich an das Unterteil anschließenden Prothesenkomponenten von dem Kopfbereich wegerstrecken, um so eine Fixierung an dem Unterteil oder der Prothesenkomponente zu ermöglichen. Die Streben oder Streifen sind vorteilhafterweise einstückig mit dem Kopfteil ausgebildet und erstrecken sich vorzugsweise medial und lateral zu dem Unterteil. Durch diese Ausgestaltung liegt das erste Rahmenteil nicht an Stellen extremer Belastungen, im Kniebereich, so dass keine hohen Kräfte beispielsweise beim Niederknien oder beim Aufstützen über das Rahmenteil aufgenommen werden müssen. Dadurch kann das Rahmenteil sehr leicht bauend ausgestaltet werden.

Das erste Rahmenteil ragt proximal über die Schwenkachse hinaus, um die natürliche Erscheinungsform im eingebeugten Zustand zu erzeugen. Darüber hinaus wird durch den Überstand ein mechanischer Schutz der dahinterliegenden Prothesenkomponenten bereitgestellt.

Das zweite Rahmenteil kann zumindest ein Überbrückungselement oder zumindest einen Überbrückungsabschnitt aufweisen, wobei sich das Überbrückungselement oder der Überbrückungsabschnitt in Richtung auf einen Prothesenschaft erstreckt, mit dem die Prothese an dem Stumpf festgelegt wird. Häufig existiert zwischen dem Prothesenschaft und dem Oberteil ein Freiraum, der über ein Verbindungsstück, z.B. ein Rohr, ein Drehelement oder Drehadapter oder dergleichen überbrückt wird. Das Verbindungsstück wird an dem proximalen Ende über einen Rohradapter an dem Prothesenschaft und an seinem distalen Ende über einen korrespondierenden Adapter an dem Oberteil des Prothesengelenkes fixiert. Das Verbindungsstück weist einen geringeren Durchmesser als der Prothesenschaft auf, so dass bei einem kosmetischen Überzug im Falle einer Einbeugung eine Konturveränderung dahingehend erfolgt, dass sich eine Delle an der Oberseite bildet. Um dies zu verhindern, wird ein Überbrückungsabschnitt an dem Rahmen ausgeformt, der sich beispielsweise bis zu dem Prothesenschaft erstrecken kann.

Der Überbrückungsabschnitt kann an dem zweiten Rahmenteil ausgebildet sein. Ein Überbrückungselement ist bevorzugt an dem zweiten Rahmenteil befestigt und erstreckt sich bis an den Prothesenschaft oder bis in dessen unmittelbarer Nähe. Es ist auch möglich, dass das Überbrückungselement und/oder der Überbrückungsabschnitt an dem Prothesenschaft festlegbar sind. Dazu weist das dem Rahmenteil abgewandte Ende des Überbrückungselementes oder des Überbrückungsabschnittes eine entsprechende Einrichtung auf, beispielsweise ein Formschlusselement oder eine kraftschlüssige Verbindungseinrichtung wie Magnete, Haftbereiche oder dergleichen. Als Formschlusselemente können Druckknöpfe, Haken, Klipse, Schrauben oder auch Niete an dem Prothesenschaft und an dem Überbrückungselement oder Überbrückungsabschnitt angeordnet sein.

Der Überbrückungsabschnitt bzw. das Überbrückungselement kann drehbar an dem zweiten Rahmenteil oder dem Prothesenschaft gelagert sein. Die drehbare Lagerung ermöglicht eine nachträgliche Ausrichtung der Prothesenkosmetik bzw. der Rahmenteile relativ zu dem Prothesenschaft oder einem Überzug. Die drehbare Lagerung kann über einen Drehadapter realisiert werden, der sowohl eine Drehung um die Längsachse des Oberteils als auch eine Verkippung beispielsweise um eine Horizontalachse zulässt. Dadurch ist es möglich, Änderungen im Aufbau des Prothesenkniegelenkes zu folgen. Über die Ermöglichung einer Drehbewegung und einer Kippbewegung ist es möglich, eine ungestörte Drehbewegung und Ausrichtung zwischen dem Prothesenschaft und dem Gelenk zu ermöglichen, ohne dass eine Störung der Einstellung des Prothesenaufbaus vorliegt. Durch die Drehbewegung und vorteilhafterweise auch eine Kippbewegung wird eine bei Veränderung des Prothesenaufbaus auftretende Verschiebung der Koaxialität der vertikalen Gelenksache und der Drehachse des Drehadapters wieder hergestellt.

An dem ersten Rahmenteil kann eine Aufnahmeeinrichtung für ein konturbildendes Formteil angeordnet sein, insbesondere kann ein Schaumteil oder ein Formteil aus einem flächigen Material an dem unteren, ersten Rahmenteil befestigt sein, um die Kontur der Prothesenkosmetik über eine Gestaltung des Rahmenteils zu individualisieren.

Eine Weiterbildung der Erfindung sieht vor, dass der Kopfbereich als separates Bauteil ausgebildet ist, das gesondert an dem Rahmenteil fixierbar ist. Dadurch können unterschiedliche Formgebungen oder Größen bereitgestellt werden, so dass ein modularer Aufbau der jeweiligen Rahmenteile erreicht werden kann. Dies erhöht die Annäherung an die natürliche Erscheinungsform.

Über die Rahmenteile wird vorteilhafterweise ein textiler Überzug gezogen, um die Rahmenteile zu überdecken. Der textile Überzug hat den Vorteil, dass keine oder nur äußerst geringe mechanische Kräfte auf das Prothesengelenk übertragen werden. Eine aufwendige Aufschrumpfung oder ein aufwendiges Aufkleben von hautähnlichen Folien auf einen Schaumkörper ist nicht mehr notwendig, da über die Rahmenkonstruktion ein formgebender Unterbau bereitgestellt wird, der nur noch ähnlich einem Strumpf mit einer quasi-geschlossenen Oberfläche versehen werden muss. Die textilen Gestaltungen sind vielfältig, ein hautfarbiger Überzug ähnlich einer Strumpfhose, kann ebenso vorgesehen sein wie andersfarbige Materialien. Der textile Überzug kann über Formschlusselemente oder über Kraftschlusselemente wie Gummiband, Klebeelemente oder Haftbereiche an dem Rahmen und/oder der weiteren Prothesenkomponente festgelegt werden. Die Festlegung kann entweder nur an dem Rahmen oder auch an der Prothesenkomponente, wie Prothesenschaft oder einem distalen Prothesenelement, wie einer Prothesenhand oder einem Prothesenfuß, erfolgen. An der jeweiligen Prothesenkomponente sind korrespondierende Einrichtungen vorgesehen, damit der textile Überzug an ihnen festgelegt werden kann. Bei einer Ausgestaltung der Formschlusselemente als Klettverschlüsse sind korrespondierende Flausch- oder Hakenbereiche in den jeweiligen Fixierbereichen vorgesehen.

In dem textilen Überzug können partielle Materialverdichtungen, Versteifungen oder Verstärkungen angebracht oder eingearbeitet sein, um neben einer reinen Oberflächenausbildung auch eine konturbildende Funktion sowie eine funktionale Komponente bereitzustellen.

Statt eines textilen Überzuges könne auch andere Überzugmaterialien verwendet werden.

Alternativ oder ergänzend kann an der Innenseite der Rahmenteile eine Beschichtung aus einem elastischen Material, beispielsweise Gummi, angebracht oder aufgebracht werden, um Schwingungen zu dämpfen oder zu verhindern, die bei einer Verwendung eines steifen Rahmenmaterials entstehen und die auf das Kniegelenk übertragen werden können. Damit kann eine Geräuschentwicklung, das sogenannte Dröhnen, verringert oder vermieden werden. Durch eine Beschichtung kann der Rahmen und dessen Lackierung geschützt werden.

Über Fixier- oder Abstandselemente kann das Rahmenteil an der Prothese befestigt oder fixiert oder relativ zu der Prothese beabstandet gehalten werden. Als Fixierelement kann beispielsweise ein Gurt oder ein flexibles Zugmittel vorgesehen sein, als Abstandselement können beispielsweise eine Metallbügel mit einem Kunststoffteil, Scheiben, Stifte oder Vorsprünge, gegebenenfalls auch als elastische Komponenten ausgebildet, vorgesehen sein, um eine ausreichende Volumenstabilität oder eine Lagestabilität des Rahmenteils relativ zu der darunterliegenden Prothesenkomponente, beispielsweise einem Rohr oder einem Schaft, bereitzustellen. Bei einer Ausgestaltung des Abstandselementes als Metallbügel wird dieser bevorzugt in dem Oberteil befestigt und ist gegen eine Verdrehung gesichert.

Die Rahmenteile bestehen bevorzugt aus formstabilen Materialien, insbesondere Metall, faserverstärkten Kunststoffen, Duroplasten oder Thermoplasten oder aus einem Schaummaterial, so dass neben der reinen Formgebung auch mechanische Belastungen aufgenommen werden können.

Nachfolgend werden Ausführungsbeispiele der Erfindung anhand der beigefügten Figuren näher erläutert. Gleiche Bezugszeichen bezeichnen gleiche Komponenten. Es zeigen:
- Figuren 1a bis 1c: eine schematische Darstellung einer Prothesenkosmetik in verschiedenen Ansichten;
- Figur 2: eine schematische Darstellung einer Prothese mit Prothesenkosmetik in Seitenansicht;
- Figur 3: eine Variante der Figur 2 mit Textilüberzug;
- Figur 4: eine Prothese gemäß Figur 3 ohne Textilüberzug;
- Figur 5: eine schematische Ansicht eines ersten Rahmenteils;
- Figur 6: eine schematische Ansicht einer Prothese nach Figur 5 mit einem zweiten Rahmenteil;
- Figur 7: eine Frontalansicht einer Prothesenkosmetik ohne Textilüberzug in gestreckter Stellung;
- Figur 8: eine Prothese in eingebeugter Stellung in Seitenansicht;
- Figur 9: montierte Rahmenteile in Extensionsstellung mit Drehadapter in Seitansicht;
- Figur 10: eine Detailansicht des zweiten Rahmenteils mit Fixierelement und Mitnehmer;
- Figur 11: Mitnehmer und Halter in Detaildarstellung;
- Figur 12: Einzeldarstellung von Abstandselementen; sowie
- Figur 13: eine perspektivische Seitenansicht einer Variante der Erfindung.

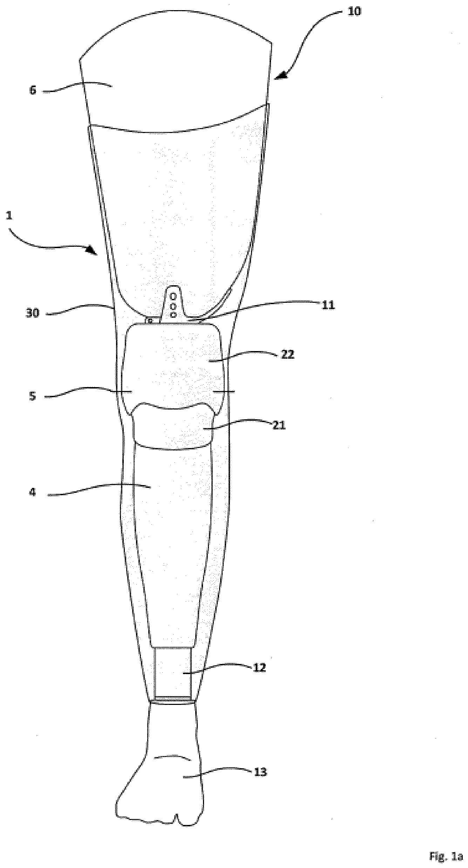

Figur 1a zeigt in einer schematischen Frontalansicht eine Prothesenkosmetik 1 für eine Prothese 10, im dargestellten Ausführungsbeispiel für ein Prothesenbein mit einem Prothesenschaft 6 zur Festlegung an einem nicht dargestellten Oberschenkelstumpf. Die Prothese 10 weist ein Prothesengelenk 2 in Gestalt eines Prothesenkniegelenkes auf, das ein Oberteil 3 sowie ein daran um eine Schwenkachse 5 schwenkbar gelagertes Unterteil 4 aufweist. An dem Unterteil 4 ist ein Prothesenfuß 13 an einem Unterschenkelrohr 12 befestigt und bildet den distalen Abschluss der Prothese 10. Statt eines Prothesenkniegelenkes 2 und eines Prothesenfußes 13 kann die Prothesenkosmetik 1 auch in einer Armprothese angeordnet und ausgebildet sein, statt eines Oberschenkelschaftes ist dann ein Oberarmschaft, statt eines Prothesenknieglenkes ein Ellenbogengelenk und statt eines Prothesenfußes eine Prothesenhand vorgesehen. Die nachfolgenden Ausführungen gelten entsprechend auch für eine Prothese der oberen Extremität.

Das Oberteil 3 des Prothesenkniegelenkes 2 ist über ein Verbindungsstück 11, beispielsweise über ein Drehadapter oder ein Rohr, mit dem Oberschenkelschaft 6 verbunden. An der Außenseite des Oberschenkelschaftes 6 ist ein Überzug 30, im dargestellten Ausführungsbeispiel ein textiler Überzug 30 angeordnet, der sich über den Gelenkbereich und über die Schwenkachse 5 bis zum proximalen Ende des Prothesenfußes 13 erstreckt. Der Überzug 30 kann formschlüssig oder kraftschlüssig an der jeweiligen Prothesenkomponente, also im dargestellten Ausführungsbeispiel dem Prothesenschaft 6 und dem Prothesenfuß 13 festgelegt werden, beispielsweise über Magnete, Klettverschlüsse, Klipse, Schrauben, Nieten oder über Klebeelement oder Hafteinrichtungen.

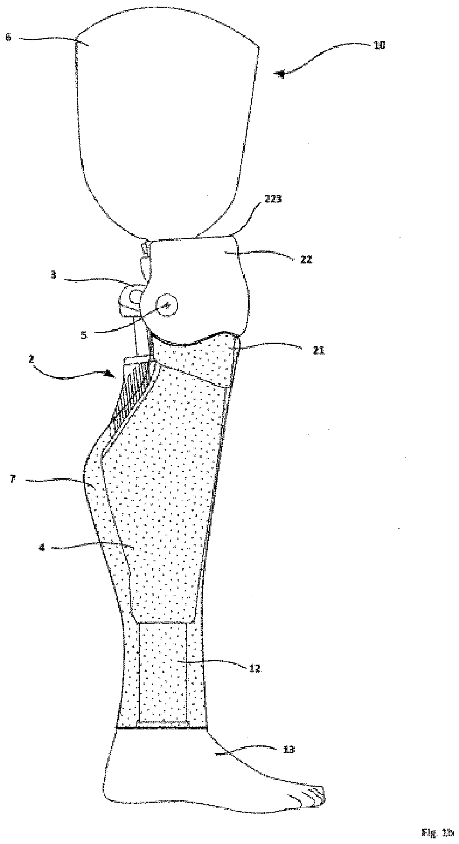

Die Prothesenkosmetik 1 sieht ein erstes Rahmenteil 21 vor, das an dem Unterteil 4 festgelegt ist. Das erste Rahmenteil 21 erstreckt sich proximal über die Schwenkachse 5 hinaus. An dem Oberteil 3 ist ein zweites Rahmenteil 22 angeordnet, das sich im dargestellten Ausführungsbeispiel distal über die Schwenkachse 5 erstreckt und im dargestellten Ausführungsbeispiel die proximale Kante des ersten Rahmenteils 21 überdeckt. An dem zweiten Rahmenteil 22 ist ein sich in proximaler Richtung erstreckender Überbrückungsabschnitt 223 ausgebildet, der sich in Richtung auf den Prothesenschaft 6 erstreckt, um eine annähernd gleichmäßige, möglichst übergangslose Kontur von dem Oberschenkelschaft 6 bis zu dem Prothesenkniegelenk 2 zu erreichen. Der Überbrückungsabschnitt 223 ist in der Figur 1b dargestellt, die eine Seitenansicht der Prothese 10 ohne Überzug 30 zeigt. Neben einem einzigen Überbrückungsabschnitt 223 können auch mehrere Überbrückungsabschnitte 223 an dem zweiten Rahmenteil 22 angeordnet oder ausgebildet sein, beispielsweise als Laschen, Zungen oder Vorsprünge. Durch den oder die Überbrückungsabschnitte 223 wird der Durchmesserunterschied zwischen dem Außendurchmesser des Prothesenschaftes 6 und dem des Verbindungsstückes 11 zur Verbindung des Prothesenschaftes 6 mit dem Prothesenkniegelenk 2 kaschiert.

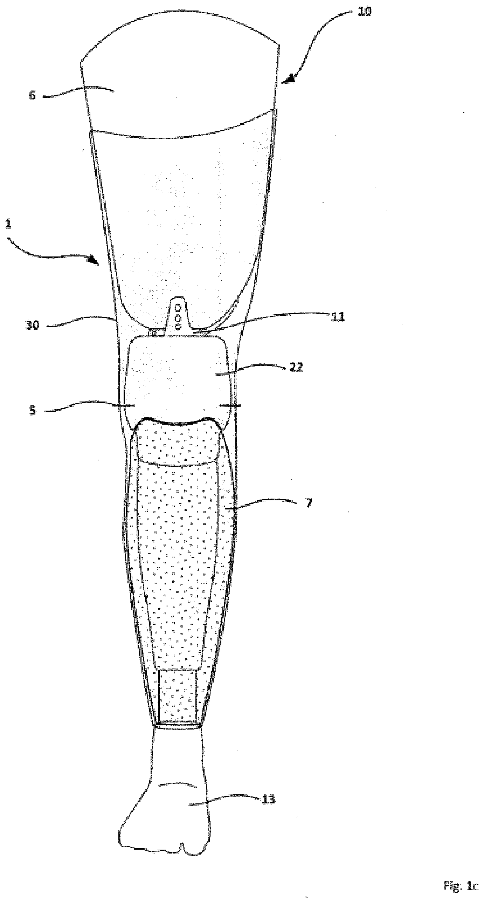

Das Prothesenkniegelenk 2 kann neben Einrichtung zum Anschluss von proximalen oder distalen Prothesenkomponenten wie Prothesenschaft 6 und Unterschenkelrohr 12 auch Dämpferelemente, Aktuatoren sowie mechanische oder elektrische und elektronische Steuerkomponenten aufweisen. Im Ausführungsbeispiel gemäß der Figur 1b ist das Unterteil 4 mit einem Formteil 7 verkleidet, das der Form eines natürlichen Unterschenkels nachgebildet ist. Das Formteil 7 kann aus einem Schaummaterial bestehen und mit einem Überzug30 verkleidet sein, wie in der Figur 1c in Frontalansicht gezeigt ist. Das erste Rahmenteil 21 kann frontal offen ausgebildet, so dass im Bereich des natürlichen Schienenbeinkopfes ein Fenster ausgebildet ist, so dass bei einer frontalen Belastung des Prothesenkniegelenkes 2, beispielsweise beim Hinknien, keine mechanische Belastung auf das erste Rahmenteil 21 aufgebracht wird.

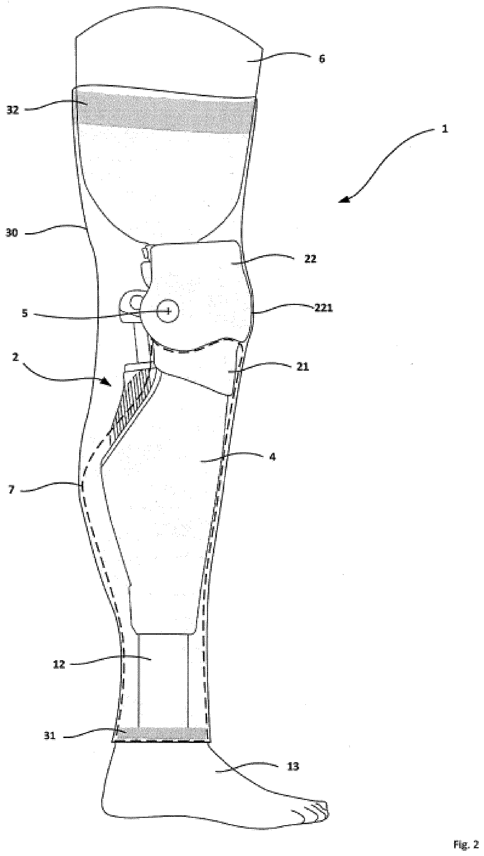

Figur 2 zeigt in einer Seitenansicht eine Variante der Erfindung, bei der die Prothesenkosmetik 1 an einem Prothesenbein in gestreckter Stellung gezeigt ist. Der Überzug 30 ist über ein Kraftschlusselement 32, im vorliegenden Fall ein elastisches Band mit einer haftenden Innenseite, an der Außenseite des Prothesenschaftes 6 festgelegt. Alternativ kann statt eines Bandes 32 ein Bereich mit einer haftenden Beschichtung, z.B. aus Silikon auf der Innenseite des Überzuges 30 angebracht sein. Der Prothesenschaft 6 ist mit dem Prothesenkniegelenk 2 über ein teilweise dargestelltes Verbindungselement in Gestalt eines Drehadapters gekoppelt. An dem Oberteil des Prothesengelenkes 2 ist das zweite Rahmenteil 22 befestigt, so dass bei einer Bewegung des Prothesenschaftes 6 das zweite Rahmenteil 22 mitgeführt wird.

An dem distalen Ende des zweiten Rahmenteils 22 ist ein Kopfbereich 221 ausgebildet, der teilkugelförmig oder teiltonnenförmig ausgebildet ist und so im Wesentlichen der natürlichen Form eines Kniegelenkes entspricht. An dem Unterteil 4 des Prothesengelenkes 2 ist das erste Rahmenteil 21 festgelegt, so dass bei einer Verschwenkung um die Schwenkachse 5 das erste Rahmenteil 21 zusammen mit dem Unterteil 3 und den sich daran anschließenden Prothesenkomponenten, beispielsweise dem Prothesenfuß 13 oder einem Unterschenkelrohr 12, mitbewegt wird. An dem ersten Rahmenteil 21 sind im posterioren Bereich ein Formteil 7 oder mehrere Formteile 7 zur Nachbildung der natürlichen Wadenmuskulatur befestigt. Das distale Ende des Überzuges 30 ist über ein Formschlusselement 31 in Gestalt eines Klettverschlusses an dem Prothesenfuß 13 festgelegt. Grundsätzlich besteht auch die Möglichkeit über andere Verbindungselemente den Überzug 30 an dem Prothesenfuß 13, dem Unterschenkelrohr oder dem Rahmenteil 21 festzulegen und zu fixieren, beispielsweise über die oben beschriebenen Kraftschlusselemente oder einen Bereich mit einer haftenden Beschichtung auf der Innenseite des Überzuges 30. Der Textilüberzug 30 erstreckt sich von dem Oberschenkelschaft 6 bis zum proximalen Ende des Prothesenfußes 13.

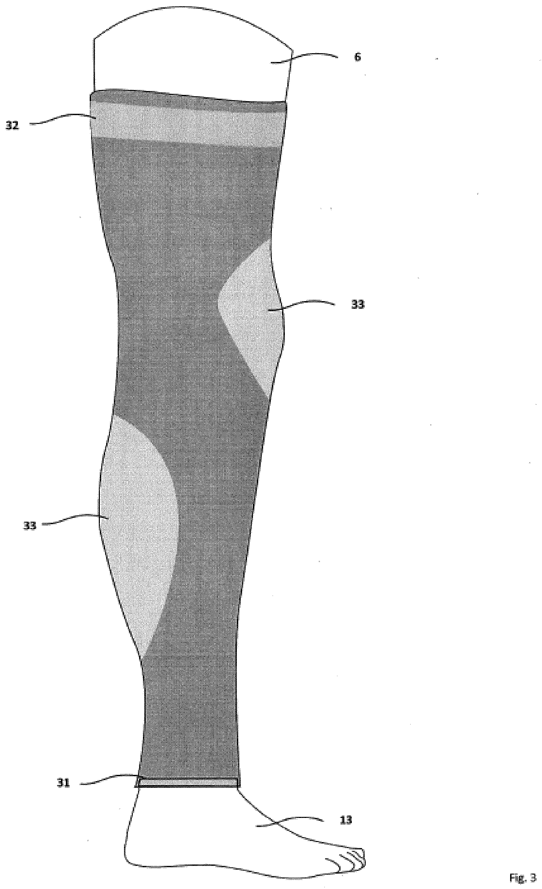

In der Figur 3 ist in einer Seitenansicht die Prothese 10 mit einer Prothesenkosmetik in Verbindung mit dem textilen Überzug 30 dargestellt. Der Textilüberzug 30 wird in Kombination mit den beiden Rahmenteilen 21, 22 verwendet und ermöglicht ein leichtes Anziehen und Ausziehen des Überzuges. Aufgrund der textilen Ausgestaltung lässt sich der Überzug 30 leicht an den Prothesenkomponenten 6, 13 und/oder den Rahmenteilen 21, 22 anbringen. Sofern ein Formteil 7 an dem Unterteil 4 angeordnet ist, kann der textile Überzug 30 an dem Formteil 7 bevorzugt über einen Silikon-Haftschluss, also eine zumindest teilweise Silikonbeschichtung an dem Überzug 30 und/oder dem Formteil 7 gehalten werden. Der Nutzer der Prothesenkosmetik 1 bekommt somit die Möglichkeit, verschiedene Designs zu verschiedenen Anlässen auszuwählen und anzulegen. Als Basisfarbe wird ein der Hautfarbe angenähertes Design vorgeschlagen. Der Überzug 30 kann einfach über die gesamt Prothese 10 gezogen werden, ebenso kann der Überzug 30 an seiner Längserstreckung offen ausgebildet und mit Verschlusselementen ausgestattet sein, beispielsweise einem Reißverschluss oder einem Klettverschluss. Eine Innenbeschichtung mit einem Elastomer oder einem Silikon verhindert ein Rutschen auf der Oberfläche der Prothese sowie der Rahmenkonstruktion. Über ein Adapterteil kann der textile Überzug 30 auch direkt in einer Fußhülle angebunden werden, so dass der Textilüberzug ohne Fußteil genutzt werden kann und bei Bedarf mit einem sockenartigen Überzug für den Prothesenfuß 13 gekoppelt werden kann. An dem Überzug sind Versteifungen 33, partielle Materialverdichtungen oder Verstärkungen angeordnet, im dargestellten Ausführungsbeispiel im Bereich des Knies und der Wade. Es können auch Versteifungen oder Verstärkungen innerhalb des Überzuges 30 angeordnet sein.

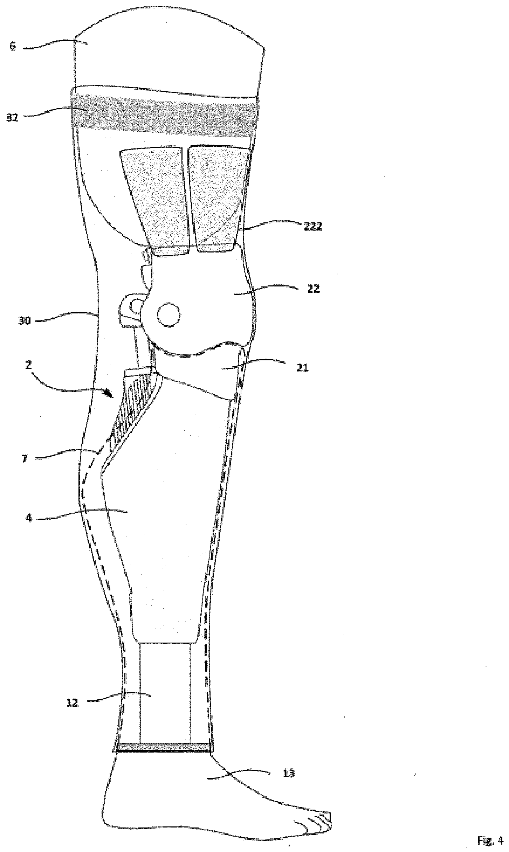

Figur 4 zeigt in einer Seitenansicht die Ausgestaltung der Prothese 10 und Prothesenkosmetik mit dem Überzug 30. Der Prothesenschaft 6 ist über das Verbindungselement 11 mit dem Prothesenkniegelenk 2 verbunden. Das zweite Rahmenteil 22 ist über nicht dargestellte Befestigungselement an dem Oberteil 3 befestigt. Von dem zweiten Rahmenteil 22 erstrecken sich Überbrückungselemente 222 in Gestalt von Gurten, Platten oder Laschen bis zu dem Prothesenschaft 6. Die Überbrückungselemente 222 sind an dem Prothesenschaft 6 formschlüssig, stoffschlüssig oder kraftschlüssig lagefixiert. Unterhalb des zweiten Rahmenteils 22 ist das erste Rahmenteil 21 angeordnet, das an dem Unterteil 4 festgelegt ist. Die Festlegung erfolgt einerseits im Bereich der Schwenkachse 5 und andererseits im distalen Bereich an dem Unterschenkelrohr 12, beispielsweise durch Klammern oder Schnappverbindungen. Die Befestigung an dem Unterschenkelrohr 12 kann auch anders erfolgen. Im distalen Bereich des ersten Rahmenteils 21 ist ein Formteil 7 zu erkennen, das der Form der natürlichen Wade nachgebildet und im dargestellten Ausführungsbeispiel aus einem Flachmaterial ausgebildet ist, das eine entsprechende Form aufweist. Alternativ dazu kann das Formteil 7 auch aus einem Schaumelement ausgebildet sein, das an dem Rahmenteil 21 über Befestigungselemente, beispielsweise Klipse, Klettverbindungen, Klebeverbindungen, Schrauben, Nieten, allgemein über Formschluss oder auch über eine Magnetbefestigung festgelegt ist.

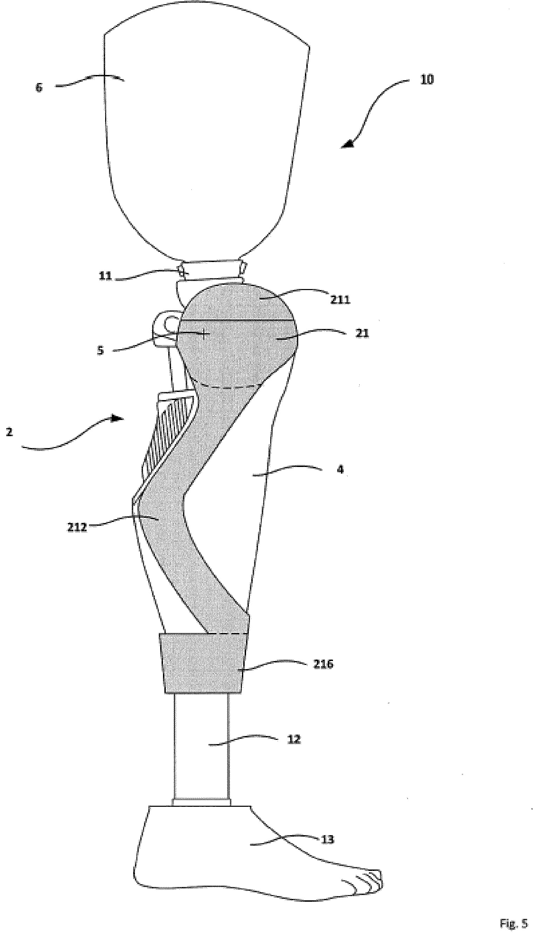

Figur 5 zeigt eine Variante des ersten Rahmenteils 21 im montierten Zustand an dem Unterteil 4 des Prothesenkniegelenkes. Das erste Rahmenteil 21 ist einstückig ausgebildet und weist einen halbkugelförmigen Kopfbereich 211 auf, der sich proximal über die Schwenkachse 5 des Prothesenkniegelenkes erstreckt. Das erste Rahmenteil 21 ist als Leichtbauteil ausgebildet und liegt eng an dem Unterteil 4 an, so dass insgesamt eine schmale Silhouette erzielt wird. In der Figur 5 ist das erste Rahmenteil 21 in Seitenansicht gezeigt, der vordere Teil sowie der hintere Teil des Unterteils 4 sind nicht von dem ersten Rahmenteil 21 überdeckt. Von dem teilkugelartigen Kopfbereich 211 erstrecken sich medial und lateral zwei Streben 212 bis zu einer distalen Spange oder Klammer 216 über die eine weitere Befestigung an dem Unterteil 4 erreicht wird. Der proximale Kopfbereich 211 weist eine Aussparung für das Verbindungselement 11 in Gestalt eines Drehadapters auf, so dass das erste Rahmenteil 21 einfach auf das Unterteil 4 aufgeschoben und über einen Schnapp- oder Klemmmechanismus daran festgelegt werden kann. Im eingebeugten Zustand bildet der keilkugelförmige Kopfbereich 211 die natürliche Knieform im Wesentlichen nach. Gemäß Figur 5 ist der frontale Bereich des Unterteils 4 frei, so dass beim Hinknien keine Kräfte in das erste Rahmenteil 21 eingeleitet werden.

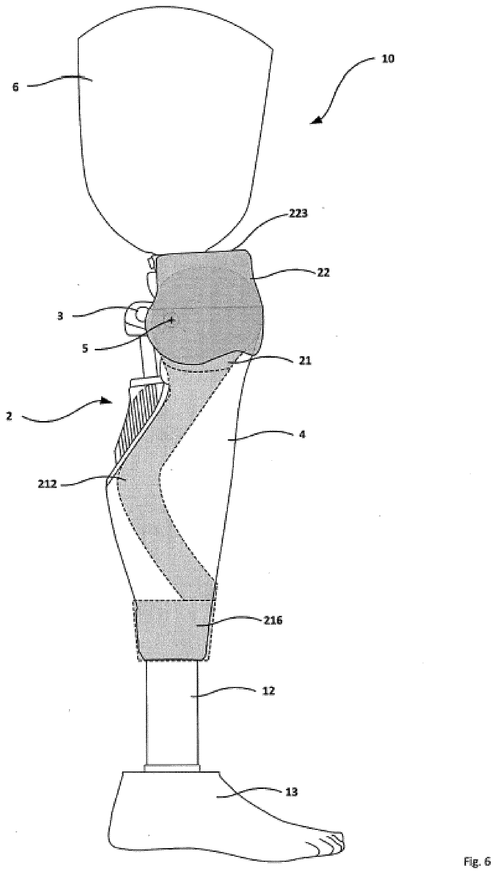

Figur 6 zeigt das zweite Rahmenteil 22 in Verbindung mit dem ersten Rahmenteil nach Figur 5, wobei das zweite Rahmenteil 22 an dem Oberteil 3 festgelegt ist, so dass es zusammen mit dem Prothesenschaft 6 bei einer Einbeugung um die Schwenkachse 5 verschwenkt wird. Das erste Rahmenteil 21 ist über Strichlinien angedeutet. Im gestreckten Zustand überdeckt das zweite Rahmenteil 22 den Kopfbereich 211 des ersten Rahmenteils, so dass sich bei einer Verschwenkbewegung der teilkugelartige Kopfbereich 211 innerhalb des zweiten Rahmenteils 22 verdreht. Der Spalt oder die Toleranzen zwischen dem ersten Rahmenteils 21 und dem zweiten Rahmenteil 22 sind möglichst gering, so dass ein Einklemmen eines Überzuges 30 in einem Zwischenraum zwischen dem ersten Rahmenteil 21 und dem zweiten Rahmenteil 22 ausgeschlossen werden kann. An dem zweiten Rahmenteil 22 sind Überbrückungsabschnitte 223 oder ein Überbrückungsabschnitt 223 ausgebildet, die sich in Richtung auf den Prothesenschaft 6 erstrecken und das Volumen zwischen einem Überzug 30 und dem Verbindungsstück 11, z.B. einem Drehadapter oder Oberschenkelrohr, ausgleichen, so dass ein Überzug 30 die Kontur einer natürlichen Gliedmaße beibehält. Das zweite Rahmenteil 22 ist frontal bevorzugt geschlossen. Ebenso ist der Kopfbereich 211 des ersten Rahmenteils 21 frontal geschlossen und weist nur einen Schlitz zur Verschwenkung des Oberteils 3 um die Schwenkachse 5 auf, so dass sowohl im Stehen als auch im gebeugten Zustand eine nahezu vollständig geschlossene Kontur im frontalen Bereich der Prothesenkosmetik 1 verwirklicht wird.

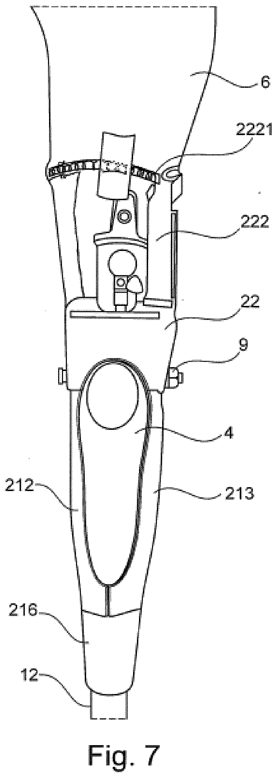

In der Figur 7 ist in einer Frontalansicht die Prothesenkosmetik 1 ohne textilen Überzug gezeigt. Das erste Rahmenteil 21 ist an dem Unterteil 4 über Befestigungselemente 9 in Gestalt von Achsen zum Stecken, Schrauben oder Muttern befestigt. An dem distalen Abschnitt erfolgt die Befestigung über einen Klickverschluss, einen Schnappverschluss oder durch andere Befestigungsmittel. Proximal der Schwenkachse 5 ist das zweite Rahmenteil 22 in einer frontal geschlossenen Formgebung angeordnet. Auch das zweite Rahmenteil 22 ist schwenkbar um die Schwenkachse 5 über die Befestigungselement 9 in Gestalt von Achsen zum Stecken an der Prothese festgelegt. Über die Überbrückungselemente 222 in Gestalt von Gewebestreifen, die über Kraftschlusselemente 2221 in Gestalt eines Silikon- oder Federbandes oder eines Gummibandes an dem Prothesenschaft 6 festgelegt sind, wird eine Kraftübertragung auf das zweite Rahmenteil 22 bewirkt, so dass bei einer Einbeugung sowohl das zweite Rahmenteil 22 als auch die Überbrückungselemente 222 mitbewegt werden. Statt Gewebestreifen können die Überbrückungselemente 222 auch Kunststoff-, Schaum- oder Metallmaterialien verwendet werden, die in Streifenfom oder ähnlicher Form ausgebildet sein können. Innerhalb der Überprüfungselemente 222 ist ein Adapteranschluss zur Anbindung des Verbindungsstückes 11 an den Prothesenschaft 6 gezeigt.

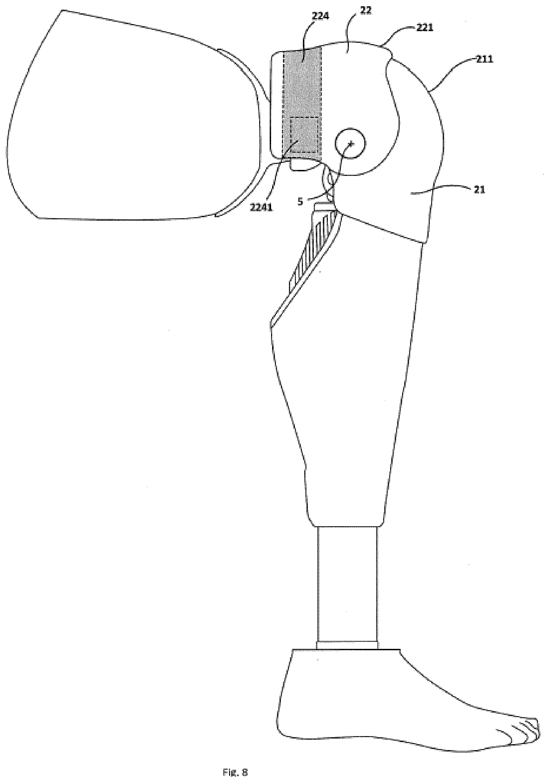

Figur 8 zeigt eine Variante der Erfindung mit einem ersten Rahmenteil 21, das als vollflächiges, schalenartiges Rahmenteil 21 ausgebildet ist. Proximal zu dem schalenartigen Schienenbeinteil schließt sich der teilkugelförmige Kopfbereich 211 an, über den der distale Kopfbereich 221 in teilkugelartiger Gestaltung des zweiten Rahmenteils 22 angeordnet ist, so dass bei einer Verschwenkung des Unterteils relativ zu dem Oberteil die Teilkalotten der Kopfbereiche 211, 221 aufeinander entlang gleiten und im eingebeugten Zustand eine sich ergänzende Teilkugel bilden. An oder in dem proximalen Endbereich des zweiten Rahmenteils 22 ist ein Drehadapter 224 angeordnet, an dem Überbrückungselemente 222 oder auch Überbrückungsbereiche 223 angeordnet oder ausgebildet sein können. Der Drehadapter 224 kann eine Relativbewegung um zumindest eine Schwenkachse, insbesondere um die Längsachse des Oberteils 2 ermöglichen, um bei einer Verkippung oder Verdrehung des Prothesenschaftes 6 relativ zu dem Prothesenkniegelenk 2 das obere Rahmenteil 22 optimal positionieren zu können. Grundsätzlich ist es auch möglich, dass innerhalb der Protheseneinrichtung ein Drehadapter vorhanden ist, um eine Verdrehung und eine Verkippung des Prothesenschaftes relativ zu dem Oberteil zu ermöglichen. Um hier eine Zugänglichkeit zu gewährleisten, können in dem zweiten Rahmenteil 22 Ausschnitte 2244, Bohrungen oder Freistellungen sowie Betätigungselemente 2241 vorgesehen sein, über die der Drehadapter 224 oder der in der Protheseneinrichtung angeordnete Drehadapter erreichbar und betätigbar sind. Dadurch ist es möglich, den jeweiligen Drehadapter 224 von außen zugänglich zu machen. Das Betätigungselement 2241 dient zur Aktivierung und Deaktivierung bzw. zum Sperren und Entsperren des Drehadapters 224 bei einem Prothesengelenk mit montierten Rahmenteilen 21, 22.

Bei der Ausgestaltung gemäß Figur 8 werden auch die Merkmale der freitragenden Struktur des Rahmens sichtbar. Vorteilhaft ist dabei die Unabhängigkeit von dem verwendeten Kniegelenk sowie die Gewichtseinsparung bei dem Prothesenkniegelenk.

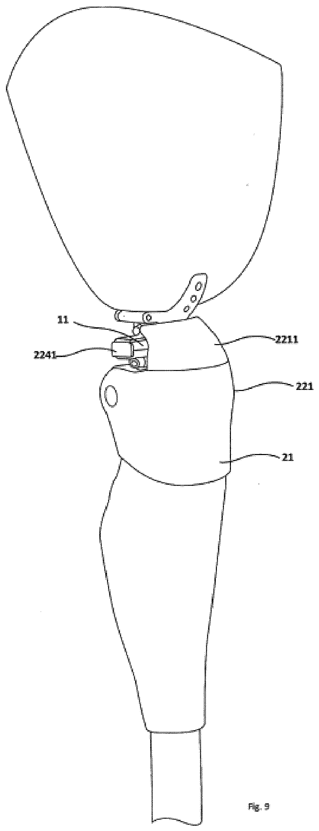

Figur 9 zeigt eine weitere Variante des ersten Rahmenteils 21, der einen Kopfbereich 211 aufweist, an dem eine Verlängerung 2211 ausgebildet oder als separates Bauteil 2211 angeordnet und an dem ersten Rahmenteil 21 befestigt ist. Die Befestigung kann durch Aufstecken, Aufklicken oder über Schrauben, Nieten oder andere Befestigungselemente erfolgen. Vorteilhafterweise kann die Verlängerung 2211 an dem Kopfbereich 221 reversibel an dem ersten Rahmenteil 21 befestigt sein. Ein wesentlicher Vorteil einer solchen modularen Lösung ist, dass damit auch lange Oberschenkelstümpfe oder Knieexartikulationsprothesen versorgt werden können. Der Kopfbereich 211 und die Verlängerung 2211 sind im Wesentlichen kugelförmig oder tonnenförmig ausgebildet, die Kugelform oder Tonnenform dient auch hier vor allem der Formgebung und dem Schutz des Überzuges vor dem Einklemmen. Die an der proximalen Kante des Kopfbereiches 211 angeordnete Verlängerung 2211 führt die teilkugelartige Kontur der Vorderkante des ersten Rahmenteils 21 nach oben hinten fort. Der Kopfbereich 211 und die Verlängerung 2211 decken zumindest in der gestreckten Stellung das Verbindungselement 11 frontal ab, bevorzugt auch, wenn das Prothesengelenk eingebeugt ist, so dass sich eine zumindest angenähert natürliche Kniekontur einstellt.

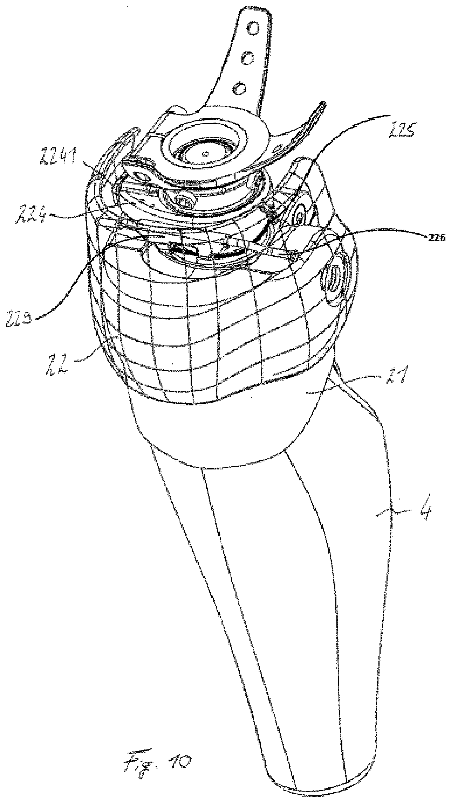

Figur 10 zeigt eine Detailansicht des Übergangsbereichs von dem zweiten Rahmenteil 22 zu einem nicht dargestellten Prothesenschaft 6. Das zweite Rahmenteil 22 umgibt das Verbindungselement zwischen dem Prothesengelenk und dem Prothesenschaft, das als Drehadapter 224 ausgebildet und an dem Prothesengelenk befestigt ist zumindest teilweise. Frontal ist der Drehadapter abgedeckt, ebenso medial und lateral, jedoch besteht ein Zugang oder eine Betätigungsmöglichkeit durch das oder mit dem zweiten Rahmenteil 22, so dass von außen der Drehadapter 224 zur Einstellung der Orientierung des Prothesenschaftes 6 zu dem Prothesenkniegelenk bzw. dem Unterteil 4 zugänglich ist. Innerhalb des zweiten Rahmenteils 22 sind ein Fixierelement 225 in Gestalt eines Bandes, Kabelbinders oder dergleichen und ein Mitnehmer 226 in Gestalt eines Bügels angeordnet, über die der Drehadapter 224 relativ zu dem zweiten Rahmenteil 22 positioniert und orientiert werden kann. Über den Mitnehmer 226 und das Fixierelement 225 wird bei einer Einbeugung des Prothesenkniegelenkes 2 das zweite Rahmenteil 22 mit dem Oberteil 3 mitgenommen.

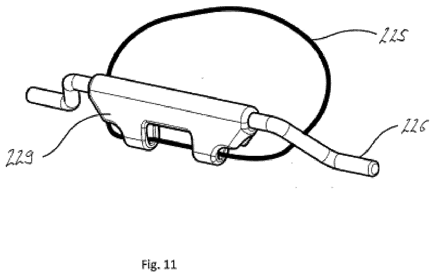

Figur 11 zeigt in einer Einzeldarstellung die Komponenten, mit denen das zweite Rahmenteil 22 gemäß Figur 10 an dem Verbindungselement 11 bzw. dem Drehadapter 224 gehalten wird. Ein Fixierelement 225 in Gestalt eines Bandes, eines Kabels, eines Kabelbinders oder eines Seils, das auch elastisch sein kann, ist in einer Aufnahme in einem Halter 229 eingelegt und daran befestigt. In dem Halter 229 ist ein Mitnehmer 226 in Gestalt eines Metallbügels drehbar gelagert. Der Mitnehmer 226 wird in dem Oberteil 3, wie in der Figur 10 dargestellt, befestigt, indem die Enden des Mitnehmers 226 in korrespondierende Ausnehmungen oder Führungen innerhalb des zweiten Rahmenteils 22 eingesteckt werden. Über das Fixierelement 225 wird der Halter 229 und damit auch der Mitnehmer 226 dicht bei dem Verbindungselement 11 bzw. dem Drehadapter 224 gehalten. Der Mitnehmer 226 wird zu seiner Justierung so lange innerhalb des Oberteils 22 verdreht, bis der Mitnehmer 226 an dem Drehadapter 224 anliegt. Dies wird dadurch erreicht, dass der Mitnehmer 226 eine abgewinkelte, kurbelartige Form aufweist, so dass durch eine Verdrehung des Mitnehmers 226 innerhalb des Oberteils 22 eine Verlagerung des Halters 229 bewirkt werden kann. In der gewünschten Position kann der Mitnehmer 226 fixiert werden, beispielsweise durch Verkleben, Verschrauben oder Einklemmen.

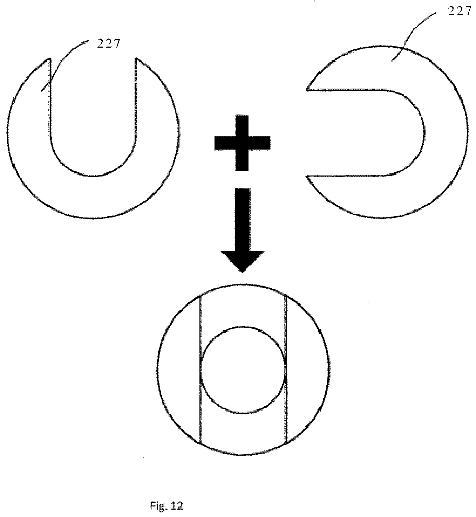

Eine weitere Möglichkeit zur Sicherstellung, dass die Bewegung des Prothesenkniegelenkes von dem zweiten Rahmenteil 22 mitgemacht wird, ist durch die Abstandselemente 227 in der Figur 12 gegeben. Die Abstandselemete 227 in Gestalt von zwei Scheiben mit Langlöchern, können um das Verbindungselement 11, beispielsweise in Gestalt eines Rohrabschnittes, herum angeordnet werden, wobei die Langlöcher oder Schlitze so zueinander verdreht werden, dass im montierten Zustand eine Verschieblichkeit entlang der jeweiligen Langlöcher nicht mehr gegeben ist. Die beiden Scheiben 227 ergänzen sich in ihrem Umfang, so dass im übereinander gelegten Zustand eine vollständig geschlossene Scheibe vorliegt.

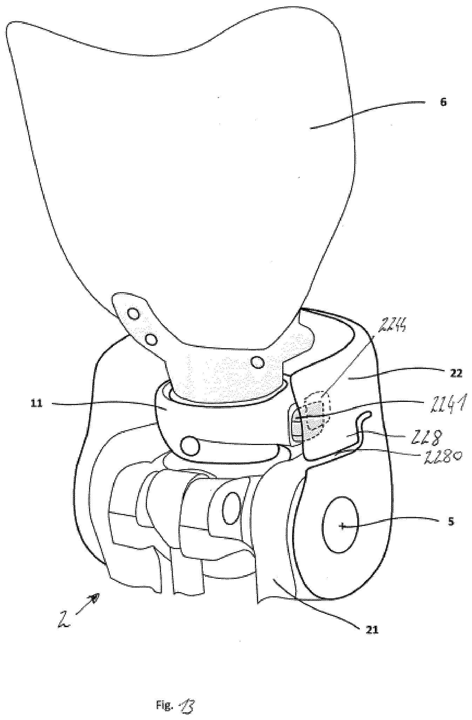

Figur 13 zeigt in einer schematischen Darstellung das Prothesenkniegelenk 2 mit einem montierten ersten Rahmenteil 21 und einem montierten zweiten Rahmenteil 22, der an dem Oberteil 3 fixiert ist, sodass bei einer Verschwenkung des Oberteils 3 mit dem Prothesenschaft 6 um die Schwenkachse 5 eine relative Verlagerung des ersten Rahmenteils 21 zu dem zweiten Rahmenteil 22 erfolgt. Im dargestellten Ausführungsbeispiel ist das Verbindungselement 11, das das Gelenkoberteil mit dem Prothesenschaft 6 verbindet, als ein Drehadapter ausgebildet, der ein Betätigungselement 2241 aufweist, das innerhalb des zweiten Rahmenteils 22 liegt. Das Betätigungselement 2241 ist von einem flexiblen Abschnitt 228 des zweiten Rahmenteils 22 seitlich abgedeckt, sodass ein direkter Zugang zu dem Betätigungselement 2241 nicht gegeben ist. Zur Betätigung des Betätigungselementes 2241, mit dem eine Verdrehung des Oberteils 3 und des Prothesenschaftes 6 relativ zu dem Unterteil ausgelöst werden kann, beispielsweise beim Hinsetzen, wird der flexible Abschnitt 228 in Richtung auf das Betätigungselement 2241 verlagert. Die Flexibilität des Abschnittes 228 wird über einen Schlitz 2280 erreicht, der innerhalb des zweiten Rahmenteils 22 ausgebildet ist, sodass ein Verlagern des flexiblen Abschnitts 228 leicht möglich ist. Der Schlitz 2280 verläuft von einem hinteren Ende des zweiten Rahmenteils 22 entlang einer seitlichen Wand, sodass ein nach innen Biegen des flexiblen Abschnitts 228 leicht möglich ist.

Sofern kein flexibler Abschnitt 228 an dem zweiten Rahmenteil 22 ausgebildet werden soll oder kann, ist alternativ eine Ausnehmung 2244 vorgesehen, die im Bereich des Betätigungselementes 2241 in dem zweiten Rahmenteil 22 ausgebildet ist. Die Ausnehmung 2244 ist in der Figur 13 durch eine strichlierte Linie angedeutet. Statt einer Ausnehmung, die vollständig von dem zweiten Rahmenteil 22 umgeben ist, kann auch ein Ausschnitt vorhanden sein, der von dem hinteren Rand des zweiten Rahmenteils 22 ausgeht. In der Figur 9 ist die Zugänglichkeit durch eine entsprechende Ausgestaltung des ersten Rahmenteils 21 gegeben.

### Bezugszeichenliste

- 1: Prothesenkosmetik
- 2: Prothesenkniegelenk
- 3: Oberteil
- 4: Unterteil
- 5: Schwenkachse
- 6: Prothesenschaft
- 7: Formteil
- 8:
- 9: Befestigungselement
- 10: Prothese
- 11: Rohr
- 12: Unterschenkelrohr
- 13: Prothesenfuß
- 21: erstes Rahmenteil
- 211: Kopfbereich
- 212: Strebe
- 213: Strebe
- 214: Schlitz
- 22: zweites Rahmenteil
- 221: Kopfbereich
- 222: Überbrückungselement
- 223: Überbrückungsabschnitt
- 224: Drehadapter
- 225: Fixierelement
- 226: Mitnehmer
- 227: Abstandselement
- 228: flexibler Abschnitt
- 229: Halter
- 30: textiler Überzug
- 31: Formschlusselement
- 32: Kraftschlusselement
- 33: Versteifung

## Patentansprüche

1. System aus einer Prothesenkosmetik (1) und einer Prothese mit einem Prothesengelenk (2), das ein Oberteil (3) und ein schwenkbar um eine Schwenkachse (5) daran gelagertes Unterteil (4) aufweist, die Prothesenkosmetik (1) weist ein erstes Rahmenteil (21), das zumindest eine erste Befestigungseinrichtung (9) zur Festlegung an dem Unterteil (4) aufweist, und ein zweites Rahmenteil (22) mit zumindest einer zweiten Befestigungseinrichtung ( 225, 226) zur Festlegung an dem Oberteil (3) auf, wobei die Rahmenteile (21, 22) schwenkbar zueinander gelagert sind,
**dadurch gekennzeichnet, dass**
an dem Prothesengelenk ein Drehelement (224) angeordnet ist, dass an einem der beiden Rahmenteile (21, 22) befestigt und an dem ein von außen betätigbares Betätigungselement (2241) angeordnet ist.

2. System nach Anspruch 1, **dadurch gekennzeichnet, dass** das Drehelement (224) als Drehadapter ausgebildet ist.

3. System nach Anspruch 1 oder 2, **dadurch gekennzeichnet, dass** das Drehelement (224) von zumindest einem Rahmenteil (21, 22) zumindest teilweise umgeben ist, wobei das Drehelement (224) durch das zumindest eine Rahmenteil (21, 22) hindurch oder mit dem zumindest einem Rahmenteil (21, 22) betätigbar ist.

4. System nach einem der voranstehenden Ansprüche, **dadurch gekennzeichnet, dass** das Drehelement (224) an dem zweiten Rahmenteil (22) und/oder an dem Oberteil (3) angeordnet ist.

5. System nach einem der voranstehenden Ansprüche, **dadurch gekennzeichnet, dass** zumindest eines der beiden Rahmenteile (21, 22) einen zumindest teilkugelartig oder teiltonnenartig ausgebildeten Kopfbereich (211, 221) aufweist, der in dem anderen Rahmenteil (22, 21) drehbar gelagert ist.

6. System nach Anspruch 5, **dadurch gekennzeichnet, dass** sowohl an dem ersten Rahmenteil (21) als auch an dem zweiten Rahmenteil (22) ein teilkugelartiger oder teiltonnenartiger Kopfbereich (211, 221) ausgebildet ist, die in einer gestreckten Stellung des Prothesengelenkes (2) einander zumindest teilweise überdecken, wobei ein Kopfbereich (211; 221) in dem anderen Kopfbereich (221; 211) aufgenommen ist.

7. System nach Anspruch 5 oder 6, **dadurch gekennzeichnet, dass** in einer eingebeugten Stellung des Prothesengelenkes (2) die Kopfbereiche (211, 221) einander ergänzend das Prothesengelenk (2) zumindest teilweise umgeben.

8. System nach einem der voranstehenden Ansprüche, **dadurch gekennzeichnet, dass** das erste Rahmenteil (21) schalenartig oder mit in Längserstreckung des Unterteils (4) gerichteten Streben (212, 213) ausgebildet ist.

9. System nach einem der voranstehenden Ansprüche, **dadurch gekennzeichnet, dass** das erste Rahmenteil (21) proximal über die Schwenkachse (5) übersteht.

10. System nach einem der voranstehenden Ansprüche, **dadurch gekennzeichnet, dass** das zweite Rahmenteil (22) zumindest ein Überbrückungselement (222) oder einen Überbrückungsabschnitt (223) aufweist, das oder der sich in Richtung auf einen Prothesenschaft (6) erstreckt.

11. System nach Anspruch 10, **dadurch gekennzeichnet, dass** das Überbrückungselement (222) oder der Überbrückungsabschnitt (223) an dem Prothesenschaft (6) festlegbar ist.

12. System nach Anspruch 10 oder 11, **dadurch gekennzeichnet, dass** das Überbrückungselement (222) oder der Überbrückungsabschnitt (223) drehbar relativ zu dem Kopfabschnitt (221) oder dem Prothesenschaft (6) gelagert ist.

13. System nach einem der voranstehenden Ansprüche, **dadurch gekennzeichnet, dass** an dem ersten Rahmenteil eine Aufnahmeeinrichtung (215) für ein konturbildendes Formteil (7) angeordnet ist.

14. System nach einem der voranstehenden Ansprüche, **dadurch gekennzeichnet, dass** der Kopfbereich (211, 221) als separates Bauteil ausgebildet ist.

15. System nach einem der voranstehenden Ansprüche, **gekennzeichnet durch** einen textilen Überzug (30), der die Rahmenteile (21, 22) überdeckt.

16. System nach Anspruch 15, **dadurch gekennzeichnet, dass** der textile Überzug (30) Formschlusselemente (31) oder Kraftschlusselemente (32) aufweist, über die der Überzug (30) an einem Rahmenteil (21, 22) und/oder einem Prothesenschaft (6) und /oder einem Schaumteil und/oder einer Fußhülle festlegbar ist.

17. System nach Anspruch 15 oder 16, **dadurch gekennzeichnet, dass** in oder an dem textilen Überzug (30) Versteifungen (33) angeordnet sind.

18. System nach einem der voranstehenden Ansprüche, **dadurch gekennzeichnet, dass** das Rahmenteil (21, 22) Fixier- oder Abstandselemente (225, 226) aufweist, über die das Rahmenteil (21, 22) an der Prothese oder relativ zu der Prothese beabstandet gehalten werden.

19. System nach einem der voranstehenden Ansprüche, **dadurch gekennzeichnet, dass** das Rahmenteil (21, 22) aus einem formstabilen Material hergestellt ist.

## Claims

1. A system consisting of a prosthesis cosmetic element according to any one of the preceding claims and a prosthesis having a prosthesis joint (2) which has an upper part (3) and a lower part (4) mounted thereon in a pivotal manner about a pivot axis (5), wherein the prosthesis cosmetic element (1) includes a first frame part (21) that includes at least one first securing means (9) for fastening to the lower part (4) and a second frame part (22) that includes at least one second securing means (225, 226) for fastening to the upper part (3) wherein the frame parts (21, 22) are pivotably supported relative to each other, **characterized in that** a rotational element (224) is disposed on the prosthesis joint, that rotational element (224) is fastened to one of the two frame parts (21, 22) and has an actuating element (2241) disposed thereon that is accessible from the outside.

2. The system according to claim 1, **characterized in that** the rotational element (224) is configured as a rotary adapter.

3. The system according to claim 1 or 2, **characterized in that** the rotational element (224) is at least partially surrounded by at least one frame part (21, 22), wherein the rotational element (224) can be actuated by going through the at least one frame part (21, 22) or by means of the at least one frame part (21, 22).

4. The system according to any one of the preceding claims, **characterized in that** the rotational element (224) is disposed on the second frame part (22) and/or on the upper part (3).

5. The system according to any one of the preceding claims, **characterized in that** at least one of the two frame parts (21, 22) includes a head area (211, 221) that is at least partially configured as ball-like or barrel-like and that is rotatably supported in the other frame part (21, 22).

6. The system according to claim 5, **characterized in that** a ball-like or partially barrel-like head area (211, 221) is configured on the first frame part (21) as well as on the second frame part (22), which overlap each another at least partially when the prosthesis joint is in the extended position (2), wherein one head area (211; 221) is received inside the other head area (221; 211).

7. The system according to claim 5 or 6, **characterized in that,** in a flexed position of the prosthesis joint (2), the head areas (211, 221) surround the prosthesis joint (2) at least partially, complementing each other.

8. The system according to any one of the preceding claims, **characterized in that** the first frame part (21) is configured as shell-like or with struts (212, 213) extending in longitudinal extension of the lower part (4).

9. The system according to any one of the preceding claims, **characterized in that** the first frame part (21) proximally protrudes the pivot axis (5).

10. The system according to any one of the preceding claims, **characterized in that** the second frame part (22) includes at least one bridging element (222) or a bridging section (223) that extends in the direction of a prosthesis socket (6).

11. The system according to claim 10, **characterized in that** the bridging element (222) or the bridging section (223) can be fastened to the prosthesis socket (6).

12. The system according to claim 10 or 11, **characterized in that** the bridging element (222) or the bridging section (223) is rotatably supported relative to the head section (221) or the prosthesis socket (6).

13. The system according to any one of the preceding claims, **characterized in that** a receiving means is disposed on the first frame part (215) for the contour-forming molded part (7).

14. The system according to any one of the preceding claims, **characterized in that** the head area (211, 221) is configured as a separate component.

15. The system according to any one of the preceding claims, **characterized in that** a textile cover (30) covers the frame parts (21, 22).

16. The system according to claim 15, **characterized in that** the textile cover (30) includes positive locking elements (31) or force fitting elements (32) by means of which the cover (30) can be fastened to a frame part (21, 22) and/or a prosthetic socket (6) and/or a foam part and/or a foot cover.

17. The system according to claim 15 or 16, **characterized in that** stiffeners (33) are disposed in or on the textile cover (30).

18. The system according to any one of the preceding claims, **characterized in that** the frame part (21, 22) includes fixation and spacer elements (225, 226) by which the frame part (21, 22) is held on the prosthesis or spaced apart from the prosthesis.

19. The system according to any one of the preceding claims, **characterized in that** the first frame part (21, 22) is produced from a dimensionally stable material.

## Revendications

1. Système constitué d'un recouvrement esthétique de prothèse (1) et d'une prothèse munie d'une articulation de prothèse (2) qui comprend une partie supérieure (3) et une partie inférieure (4) montée sur celle-ci de manière à pouvoir pivoter autour d'un axe de pivotement (5), le recouvrement esthétique de prothèse (1) comprenant une première partie de cadre (21) qui présente au moins un premier dispositif de fixation (9) pour la fixation à la partie inférieure (4), et une deuxième partie de cadre (22) qui présente au moins un deuxième dispositif de fixation (225, 226) pour la fixation à la partie supérieure (3), les parties de cadre (21, 22) étant montées de manière à pouvoir pivoter l'une par rapport à l'autre,
**caractérisé en ce qu'**un élément rotatif (224) est disposé sur l'articulation de prothèse, qui est fixé à l'une des deux parties de cadre (21, 22) et sur lequel est disposé un élément d'actionnement (2241) pouvant être actionné de l'extérieur.

2. Système selon la revendication 1,
**caractérisé en ce que** l'élément rotatif (224) est conçu comme un adaptateur rotatif.

3. Système selon la revendication 1 ou 2,
**caractérisé en ce que** l'élément rotatif (224) est au moins partiellement entouré par au moins une partie de cadre (21, 22), l'élément rotatif (224) pouvant être actionné à travers ladite au moins une partie de cadre (21, 22) ou avec ladite au moins une partie de cadre (21, 22).

4. Système selon l'une des revendications précédentes,
**caractérisé en ce que** l'élément rotatif (224) est disposé sur la deuxième partie de cadre (22) et/ou sur la partie supérieure (3).

5. Système selon l'une des revendications précédentes,
**caractérisé en ce que** l'une au moins des deux parties de cadre (21, 22) présente une zone de tête (211, 221) réalisée au moins en forme de sphère partielle ou de tonneau partiel, qui est montée de manière rotative dans l'autre partie de cadre (22, 21).

6. Système selon la revendication 5,
**caractérisé en ce qu'**une zone de tête (211, 221) en forme de sphère partielle ou de tonneau partiel est formée aussi bien sur la première partie de cadre (21) que sur la deuxième partie de cadre (22), qui se recouvrent au moins partiellement l'une l'autre dans une position en extension de l'articulation de prothèse (2), une zone de tête (211 ; 221) étant logée dans l'autre zone de tête (221 ; 211).

7. Système selon la revendication 5 ou 6,
**caractérisé en ce que** dans une position en flexion de l'articulation de prothèse (2), les zones de tête (211, 221) entourent au moins partiellement l'articulation de prothèse (2) en se complétant.

8. Système selon l'une des revendications précédentes,
**caractérisé en ce que** la première partie de cadre (21) est réalisée en forme de coque ou avec des entretoises (212, 213) orientées dans le sens longitudinal de la partie inférieure (4).

9. Système selon l'une des revendications précédentes,
**caractérisé en ce que** la première partie de cadre (21) dépasse de manière proximale de l'axe de pivotement (5).

10. Système selon l'une des revendications précédentes,
**caractérisé en ce que** la deuxième partie de cadre (22) comprend au moins un élément de pontage (222) ou une portion de pontage (223) qui s'étend vers une emboîture de prothèse (6).

11. Système selon la revendication 10,
**caractérisé en ce que** l'élément de pontage (222) ou la portion de pontage (223) peut être fixé(e) à l'emboîture de prothèse (6).

12. Système selon la revendication 10 ou 11,
**caractérisé en ce que** l'élément de pontage (222) ou la portion de pontage (223) est monté(e) de manière rotative par rapport à la partie de tête (221) ou à l'emboîture de prothèse (6).

13. Système selon l'une des revendications précédentes,
**caractérisé en ce qu'**un dispositif de réception (215) pour une pièce moulée (7) formant le contour est disposé sur la première partie de cadre.

14. Système selon l'une des revendications précédentes,
**caractérisé en ce que** la zone de tête (211, 221) est conçue comme un composant séparé.

15. Système selon l'une des revendications précédentes,
**caractérisé par** un revêtement textile (30) qui recouvre les parties de cadre (21, 22).

16. Système selon la revendication 15,
**caractérisé en ce que** le revêtement textile (30) présente des éléments de coopération de forme (31) ou des éléments de coopération de force (32) permettant de fixer le revêtement (30) à une partie de cadre (21, 22) et/ou à une emboîture de prothèse (6) et/ou à une partie en mousse et/ou à une enveloppe de pied.

17. Système selon la revendication 15 ou 16,
**caractérisé en ce que** des raidisseurs (33) sont disposés dans ou sur le revêtement textile (30).

18. Système selon l'une des revendications précédentes,
**caractérisé en ce que** la partie de cadre (21, 22) présente des éléments de fixation ou d'écartement (225, 226) par lesquels la partie de cadre (21, 22) est maintenue sur la prothèse ou à distance de la prothèse.

19. Système selon l'une des revendications précédentes,
**caractérisé en ce que** la partie de cadre (21, 22) est fabriquée en un matériau indéformable.
